# EUROPEAN PATENT APPLICATION

(11) **EP 2 085 034 A1**
(43) Date of publication of application: **05.08.2009**
(21) Application number: 09001246.9
(22) Date of filing: 29.01.2009
(51) Int. Cl.: A61B 17/28, A61B 17/00

(54) **Endoscope treatment instrument**

(30) Priority: 01.02.2008 US 24387
(71) Applicant: Olympus Medical Systems Corporation, Tokyo 151-0072 (JP)
(72) Inventor: Okada, Tsutomu, Tokyo 151-0072 (JP); Miyamoto, Manabu, Tokyo (JP); Nomura, Yusuke, Tokyo 151-0072 (JP)
(74) Representative: von Hellfeld, Axel

(57) **Abstract**

An endoscope treatment instrument provided with an inserted portion that is flexible, a treatment portion that is attached to the distal end of the inserted portion, an operator portion that is attached to the proximal end of the inserted portion for manipulating the treatment portion, and a transmitting member that is connected to the treatment portion and the operator portion and is inserted into the inserted portion, for transmitting the operation of the operator portion to the treatment portion; wherein:
the operator portion has a first handle that is attached to the inserted portion so as to prevent movement in the axial direction of the inserted portion, a second handle that is attached to the first handle so as to permit rotation, a linking member in which its first end is attached to the second handle so as to permit rotation, and its second end is connected to the transmitting member, and a positional relationship maintaining mechanism which can maintain the positional relationship between the linking member and the first handle; and
when the operator portion is operated, the angle formed by the second handle and the linking member changes, transitioning to an operating state in which the second end of the linking member has moved toward the proximal end side, and this operating state is maintained by the positional relationship maintaining mechanism.

## Description

### BACKGROUND OF THE INVENTION

### Field of the Invention

The present invention relates to an endoscope treatment instrument which is employed by insertion into a body cavity.

Priority is claimed on United State Patent Application No.12/024,387, filed on February 1, 2008, the content of which is incorporated herein by reference.

### Description of Related Art

Cholecystectomy and other various procedures using laparoscopy and the like are examples of minimally invasive therapies which have been carried out conventionally. In this type of laparoscopic procedure, a plurality of openings are made in the abdominal wall and various instruments are inserted through these openings.

In recent years, designs have been proposed to carry out these procedures by inserting a flexible endoscope into the patient via a natural orifice, e.g., mouth, naris, anus, etc., with the goal of reducing the burden on the patient by decreasing the number of openings made in the abdominal wall. The medical treatment endoscope disclosed in U.S. Patent Application Publication No. 2007/0167680 may be cited as an example of a medical treatment endoscope employed in this type of procedure. This medical treatment endoscope has a flexible, soft inserted portion, and a pair of arms which are provided to the distal end of the inserted portion and have bending parts for performing bending actions. In addition, a plurality of channels are provided running through the inserted portion. A member is provided to the operator portion of the medical treatment endoscope for performing forward, backward, left and right bending manipulation of the arms. The user inserts the inserted portion of an instrument into the channel, attaches the operator portion of the instrument to the operator portion of the medical treatment endoscope, projects the distal end of the instrument out from the arm, and manipulates the operator portion to the front, back, left or right. As a result, the distal end of the instrument can be made to approach the target tissue from different directions in order to carry out the procedure.

The instruments which may be used in combination with this type of medical treatment endoscope must be flexible instruments that are equipped with a flexible sheath. The operator portion for a flexible instrument that is employed in combination with the typical flexible endoscope frequently employs a design in which the index finger is passed through the proximal end thereof, and the slider is held by the other fingers and moved back and forth.

However, when carrying out a procedure using the combination of the aforementioned medical treatment endoscope and a flexible instrument having an operator portion such as described above, it is necessary to perform manipulations by moving the handle of the medical treatment endoscope up, down, left and right while concurrently holding the operator portion of the instrument. This is problematic as manipulation of the device becomes difficult as a result.

On the other hand, a gun-grip or in-line type operator portion is frequently employed in the case of rigid instruments used in laparoscopic procedures, etc. These operator portions have the benefit of being familiar in use to the surgeons who typically carry out endoscopic procedures via a natural orifice.

However, in the case of the above-described operator portions employed in rigid instruments, the stroke required for actuating the treatment mechanism at the distal end of the instrument is small. Thus, while not a problem in the case of a rigid sheath, in the case of a flexible instrument, the sheath and the operating wire inserted into the sheath expand, so that a larger stroke is required than in the case of the rigid instrument. Thus, it is difficult to employ these operator portions for flexible instruments without modification.

### SUMMARY OF THE INVENTION

The present invention was conceived in view of the above-described circumstances, and has as its objective the provision of an endoscope treatment instrument which has excellent operability and can obtain a sufficient operating stroke.

The present invention is an endoscope treatment instrument provided with an inserted portion that is flexible, a treatment portion that is attached to the distal end of the inserted portion, an operator portion that is attached to the proximal end of the inserted portion for manipulating the treatment portion, and a transmitting member that is connected to the treatment portion and the operator portion and is inserted into the inserted portion, for transmitting the operation of the operator portion to the treatment portion; wherein:
the operator portion has a first handle that is attached to the inserted portion so as to prevent movement in the axial direction of the inserted portion, a second handle that is attached to the first handle so as to permit rotation, a linking member in which its first end is attached to the second handle so as to permit rotation, and its second end is connected to the transmitting member, and a positional relationship maintaining mechanism which can maintain the positional relationship between the linking member and the first handle; and
when the operator portion is operated, the angle formed by the second handle and the linking member changes, transitioning to an operating state in which the second end of the linking member has moved toward the proximal end side, and this operating state is maintained by the positional relationship maintaining mechanism.

### BRIEF DESCRIPTION OF THE DRAWINGS

FIG 1 is a view showing the endoscope treatment instrument according to a first embodiment of the present invention.
FIG. 2 is a view showing the main body and the inserted portion of the same endoscope treatment instrument.
FIGS. 3A and B are cross-sectional views of the operator portion of the same endoscope treatment instrument.
FIG 4 is a view showing the gripping operation for the same endoscope treatment instrument.
FIGS. 5A through C are views showing the movement of various parts of the same endoscope treatment instrument during operation.
FIG 6 is a view showing the endoscope treatment instrument according to a second embodiment of the present invention.
FIG. 7 is an exploded view of the treatment portion of the same endoscope treatment instrument.
FIG 8 is a view showing the treatment portion in the open state.
FIG 9 is an exploded cross-sectional view of the inserted portion of the same endoscope treatment instrument.
FIG 10 is a cross-sectional view of the operator portion of the same endoscope treatment instrument.
FIG 11 is an exploded cross-sectional view in the vicinity of the proximal end of the wire of the same endoscope treatment instrument.
FIG 12 is a view showing the action of the operator portion during use of the same endoscope treatment instrument.
FIG 13 is a view showing the action of the operator portion during use of the same endoscope treatment instrument.
FIG 14 is an exploded cross-sectional view of the inserted portion in a modification of the same endoscope treatment instrument.
FIGS. 15 A and B are views showing the action during use of the operator portion in a modification of the same endoscope treatment instrument.
FIG. 16 is a view showing the endoscope treatment instrument according to a first embodiment of the present invention.
FIG 17 is a cross-sectional view showing the main body and the operator portion of the same endoscope treatment instrument.
FIG. 18 is a view showing the action of the operator portion during use of the same endoscope treatment instrument.
FIG 19 is a view showing the arrangement when the present invention's endoscope treatment instrument is inserted into the operation stick of a medical treatment endoscope.

### DETAILED DESCRIPTION OF THE INVENTION

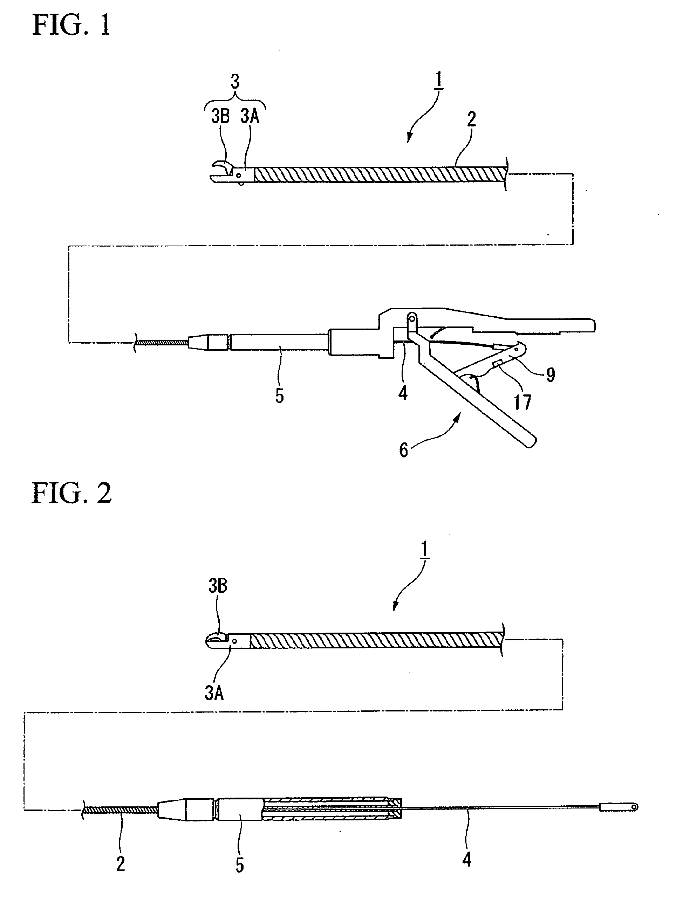

The endoscope treatment instrument according to a first embodiment of the present invention will be explained with reference to FIGS. 1 through 5C.

FIG 1 is a view showing a needle-holding device 1 which is the endoscope treatment instrument according to this embodiment. This needle-holding device 1 is a flexible treatment instrument, and is provided with a flexible inserted portion 2, a treatment portion 3 that is attached to the distal end of the inserted portion 2, a wire 4 that is connected to the treatment portion 3, a main body 5 connected to the proximal end of the inserted portion 2, and an operator portion 6 that is connected to the main body 5.

The inserted portion 2 is formed of a material that has flexibility, such as a coil sheath, and is advanced into a body cavity by insertion through the instrument channel of the endoscope.

The treatment portion 3 is a conventional design, and consists of paired jaws 3A and 3B for gripping a suturing needle. The wire 4 is connected to the jaw 3B. As will be explained below, the jaw 3 revolves by manipulation of the wire 4 via the operator portion 6, causing the treatment portion 3 to open and close, and enabling gripping of the needle.

The wire 4 is a transmitting member for transmitting the operation of the operator portion 6 to the treatment portion 3. The wire 4 is connected to the jaw 3B. As shown in FIG. 2, the wire 4 is passed through the inside of the inserted portion 2 and the main body 5, and is connected to the operator portion 6.

The main body 5 is formed of a resin or the like, and is hard. The proximal end of the inserted portion 2 is attached to the distal end of the main body 5 in a manner which does not permit rotation. When inserting the needle-holding device 1 into the endoscope, the main body 5 engages with the handle of the endoscope when the needle-holding device 1 is inserted into the endoscope, and fixes the needle-holding device 1 in place to permit advancing, retracting and rotating with respect to the endoscope.

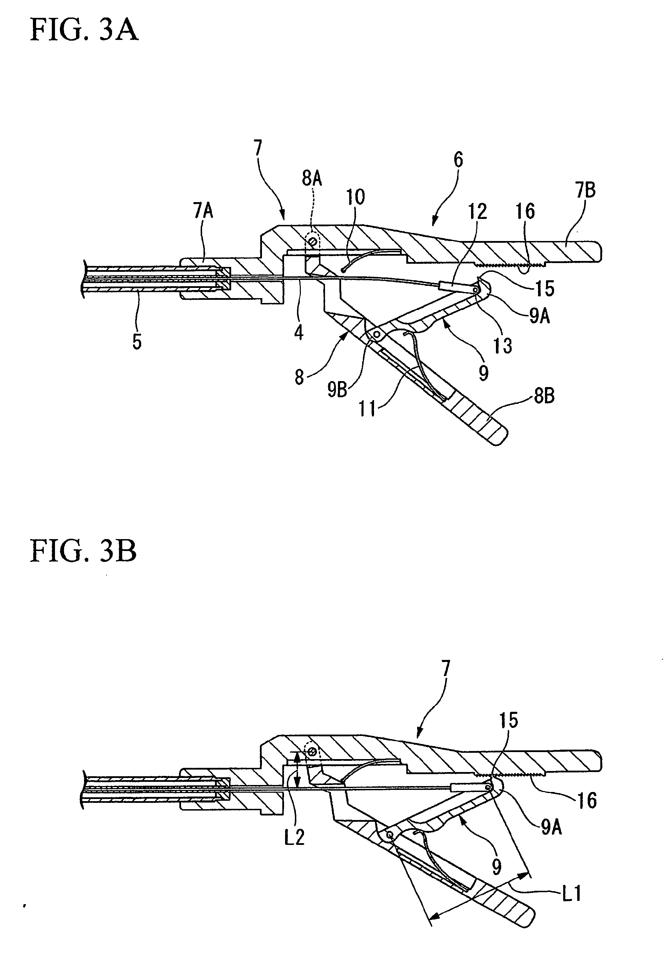

FIGS. 3A through 5C are all cross-sectional views showing the operator portion 6. The operator portion 6 is a so-called in-line type handle, and is composed of a first handle 7 that is attached to the main body 5, and a second handle 8 that is attached to permit rotation within a fixed range with respect to the first handle 7.

The distal end 7A of the first handle 7 is attached to the proximal end of the main body 5. The proximal end side of the first handle 7 composes a grip part 7B which grips when the user is operating it. The proximal end of the wire 4 which is inserted into the main body 5 extends toward the grip part 7B.

In the second handle 8, the first end 8A is fixed to permit free rotation with respect to the first handle 7. The second end 8B side forms a grip part that the user grips. The second handle 8 is biased so as to move away from the first handle 7 under the elastic force of a first biasing member 10 that is attached to the first handle 7. As a result, when the user releases the second handle 8 and releases the engagement between a claw 15 and a ratchet 16, explained further below, the first handle 7 and the second handle 8 move apart a specific distance.

A linking member 9, in which the proximal end of the wire 4 is connected to the first end 9A, is attached to the second handle 8 so as to permit rotation between the first handle 7 and the second handle 8. The second end 9B of the linking member 9 is attached in a freely rotating manner to the second handle 8, and is biased by a second biasing member 11 that is attached to the second handle 8, so as to move away from the second handle 8 by a set angle.

A connecting member 12 which is provided to the proximal end of the wire 4 is attached to the second end 9B of the linking member 9 via a pin 13. As a result, the angle formed by the wire 4 and the linking member 9 can be changed.

A claw 15 is provided to the first end 9A side of the linking member 9. As shown in FIG. 3B, this claw 15 can engage with the ratchet 16 that is provided to the first handle 7. Accordingly, when the claw 15 and the ratchet 16 engage, a constant positional relationship is maintained between the linking member 9 and the first handle 7. The engagement of the claw 15 and the ratchet 16 can be released by operating the release button 17 (see FIG 1) that is provided to the linking member 9.

The action of a needle-holding device 1 formed as disclosed above during use will now be explained.

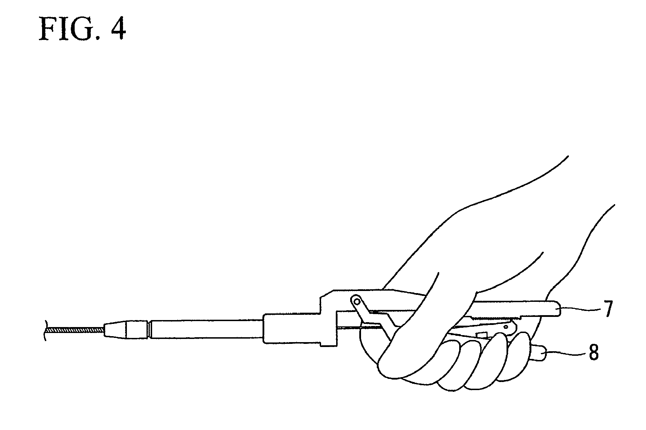

First, as shown in FIG 4, the user grips the first handle 7 and the second handle 8, bringing the handles together. As a result, the wire 4 is pulled toward the handheld side, and the jaw 3B of the treatment portion 3 rotates toward the distal end side, closing the treatment portion 3. This arrangement is maintained even if the user releases his hand, due to the engagement between the claw 15 of the linking member 9 and the ratchet 16 of the first handle 7. The movements of the various mechanisms of the operator portion 6 at this time will be explained below.

With the treatment portion 3 closed, the user inserts the treatment portion 3 and the inserted portion 2 via an opening provided in the operator portion of the medical treatment endoscope into the channel, and fixes the distal end of the main body 5 to one of the handles of the operator portion of the medical treatment endoscope. The operator portion 6 is advanced with respect to the handle of the medical treatment endoscope, and the main body 5 is pushed inside the handle, causing the treatment portion 3 to be projected into the patient's body cavity from the distal end of the arm which is projecting out from the inserted portion of the medical treatment endoscope.

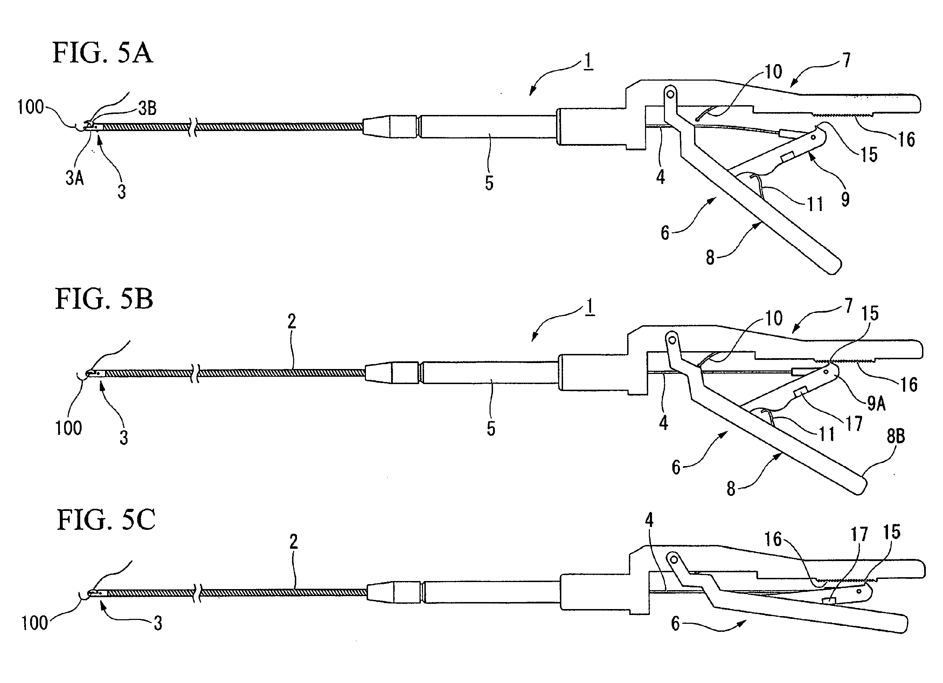

The user operates a release button 17, and releases the engagement between the linking member 9 and the first handle. As a result, as shown in FIG 5A, the first handle 7 and the second handle 8 move away from one another due to the biasing force of the first biasing member 10. The linking member 9 rotates toward the first handle 7 due to the biasing force of the second biasing member 11, and the wire 4 moves toward the main body 5 side. As a result, the distal end of the jaw 3B rotates toward the proximal end side, opening the treatment portion 3.

A curved needle 100 which is delivered into the body by a separate method, is introduced in between the paired jaws 3A and 3B. The user again grips the first handle 7 and the second handle 8, closing the treatment portion 3 and thereby gripping the curved needle 100.

FIGS. 5A through 5C are views showing the movement of the needle-holding device 1 when the first handle 7 and the second handle 8 are gripped. In the arrangement shown in FIG. 5A, which is prior to gripping of the first handle 7 and the second handle 8, the claw 15 and the ratchet 16 are not engaged and the treatment portion 3 is open.

When the handles 7,8 are gripped, then the second handle 8 moves toward the first handle 7 against the biasing force of the first biasing member 10, and the claw 15 of the linking member 9 engages with the ratchet 16 of the first handle 7, as shown in FIG. 5B. When the user applies additional force, the linking member 9 moves against the biasing force of the second biasing member 11 so that the second end 9B approaches the end 8B on the gripping side of the second handle 8.

As a result, the angle formed by the second end 9B of the linking member and the first end 8A of the second handle is gradually increased. The claw 15 rides over the crest of the ratchet 16 with which it is engaged, and engages with a crest that is farther toward the handheld side. The wire 4 is pulled toward the handheld side, and jaw 3B closes, thereby gripping the curved needle 100 in the treatment portion 3. The state of deformation of the operator portion as described above during operation of the treatment portion 3 is maintained by the positional relationship maintaining mechanism that is formed by the claw 15 and the ratchet 16.

If the user then applies further force, the claw 15 will then engage with a crest in the ratchet 16 that is farther toward the handheld side, and the wire 4 is pulled toward the handheld side, as shown in FIG 5C. As a result, the treatment portion 3 can grip the curved needle 100 with greater strength.

With the curved needle 100 held within the body cavity of the patient, the user rotates the operator portion 6, causing the inserted portion 2 and the treatment portion 3 to rotate. As a result of this manipulation, the curved needle 100 penetrates the tissue and suturing of the surgical incision, etc., is carried out. During the suturing operation, such actions as releasing and re-gripping the curved needle 100 by appropriately employing the release button 17, inserting an equivalent needle-holding device 1 via another channel in the medical treatment endoscope and transferring the curved needle 100 thereto, etc., are carried out.

In the needle-holding device 1 in the present embodiment, the proximal end of the wire 4 is connected to permit rotation to the second end 9B of the linking member 9 which is connected to the second handle 8. The angle formed by the second handle 8 and the linking member 9 changes based on the user's gripping operation, and second end 9B moves farther toward the handheld side.

Accordingly, it is possible to obtain a greater stroke as compared to the case where the proximal end of the wire 4 is connected near the first end 8A, where the second handle 8 is connected to the first handle 7, such as in the conventional rigid endoscope treatment instrument. As a result, the gripping manipulation of the curved needle 100 by the treatment portion 3 can be carried out with certainty, even if the inserted portion 2 is flexible.

Note that in order to obtain an excellent stroke, it is preferable that the length L1 between the position at which the proximal end of the wire is connected and the connecting position with the second handle 8 in the linking member 9, be set to be longer than the distance L2 between the first end 8A of the second handle and the wire 4.

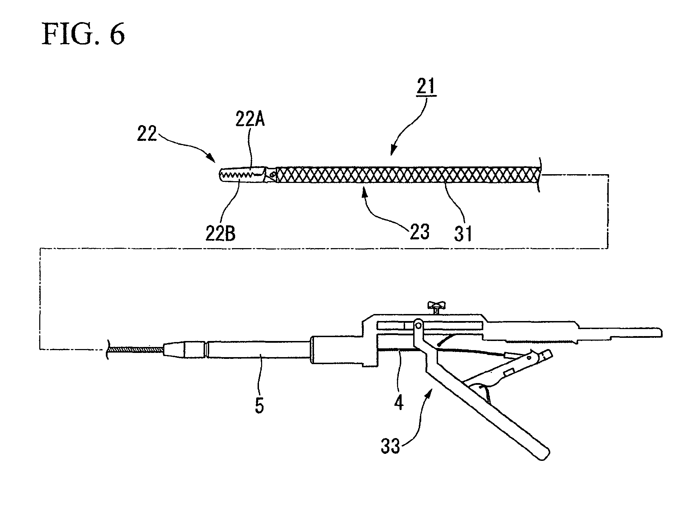

Next, the second embodiment of the present invention will be explained with reference to FIGS. 6 through 15B. The endoscope treatment instrument differs from the above-described needle-holding device 1 with respect to the structure of the treatment portion and the inserted portion, and in that the position of attachment of the second handle to the first handle can move.

Note that the compositional elements that are the same as that of the above-described needle-holding device 1 will be assigned the same numeric symbol, and a repetitive explanation thereof will be omitted.

FIG 6 is a view showing the gripping forceps 21 which is the endoscope treatment instrument in this embodiment. The treatment portion 22 of the gripping forceps 21 is designed to include a pair of forceps members 22A and 22B.

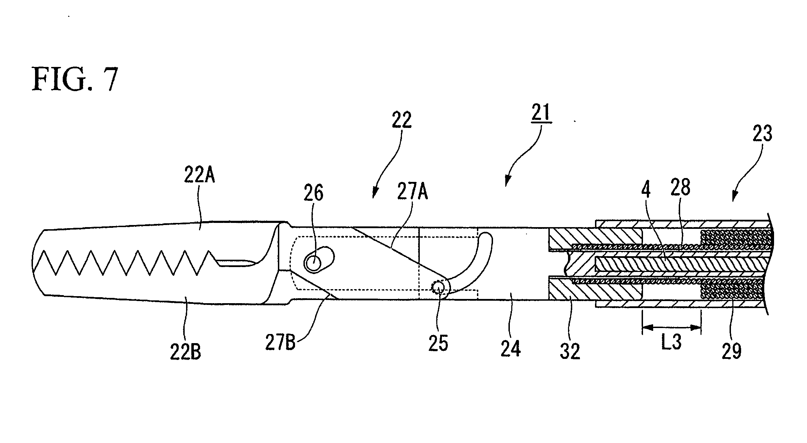

FIG 7 is an exploded view of the treatment portion 22. In this figure, the lower forceps member 22B engages with an opening/closing member 24 on its handheld side via a pin 25, and is supported by the distal end member 32 via a pin 26. The upper forceps member 22A composes roughly the same design as the forceps member 22B. The opening/closing member 24 is connected to the distal end of the wire 4. By manipulating the wire 4, the opening/closing member 24 can be advanced and retracted along the axial direction with respect to the inserted portion 23 and the distal end member 32.

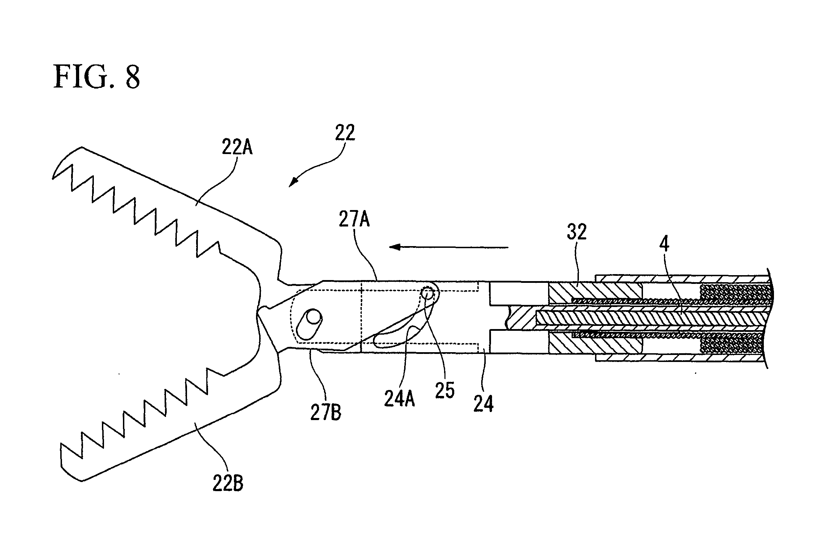

When the opening/closing member 24 is moved toward the distal end side by advancing the wire 4, then the pin 25 moves along the engagement groove 24A of the opening/closing member, as shown in FIG 8. The forceps members 22A,22B open accompanying this movement, thus opening the treatment portion 22. Inclined surfaces 27A and 27B are provided to the proximal end side of the forceps member 22B. When the treatment portion 22 is opened, the inclined surfaces 27A,27B become substantially parallel to the axis of the wire 4, and the proximal end sides of the forceps member 22B do not project out radially beyond the outermost periphery of the distal end member 32.

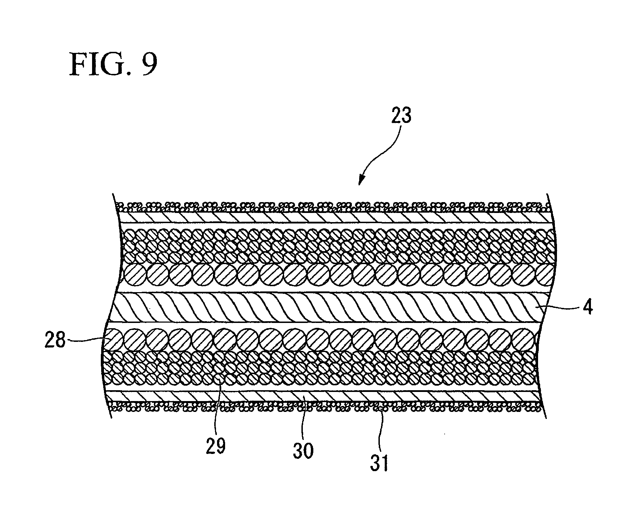

FIG. 9 is an exploded cross-sectional view of the inserted portion 23. The inserted portion 23 is provided with a first sheath 28 into which the wire 4 is inserted, a second sheath 29 which is provided to the outside of the first sheath 28, a cover tube 30 for covering the outside of the second sheath 29, and a blade 31 which is provided to the outside of the cover tube 30.

First sheath 28 is a coil sheath. The second sheath 29 is a conventionally known 3-layer coil sheath in which three layers of metal strands are placed on top of one another. The distal end of the second sheath 29 is fixed in place to the first sheath 28 by soldering or other such methods. The second sheath 29 transmits the rotation operation of the operator portion to the treatment portion 22 excellently.

Cover tube 30 consists of a resin or the like, and is flexible. The blade 31 is formed by weaving resin strands into a tube form comprising net meshing. The cover tube 30 is inserted inside the blade 31.

As shown in FIG 7, the treatment portion 22 is attached to the distal end of the first sheath 28 and the cover tube 30 via the distal end member 32. The second sheath 29 is fixed in place to the first sheath 28 at a position which is separated by a specific length L3 from the distal end member 32. The highly rigid treatment portion 22 and distal end member 32, and the distal end of the second sheath 29, which has had its rigidity increased by soldering are not connected at the distal end of the gripping forceps 21. Thus, the length of the highly rigid portion at the distal end of the gripping forceps 21 can be held to a short length.

The specific length L3 is preferably in the range of 2 to 20 millimeters. When L3 is less than 2 millimeters, it is not possible to maintain flexibility, and when L3 is greater than 20 millimeters, it is no longer possible to transmit the rotation operation to the distal end well. In this embodiment, the length of L3 is set to be 3 millimeters.

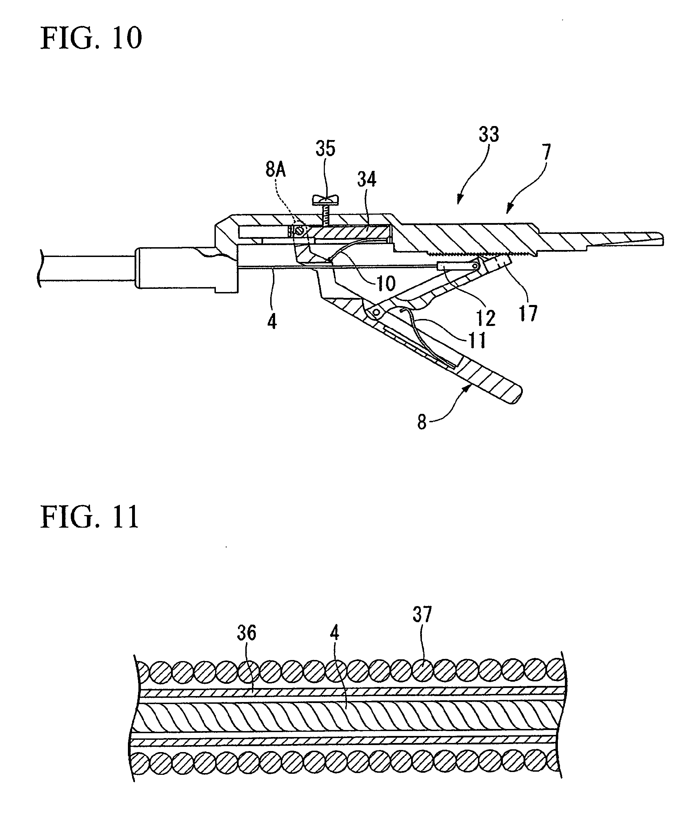

FIG. 10 is a cross-sectional view of the operator portion 33. The first end 8A of the second handle 8 and the first biasing member 10 are attached to an ejector 34 which is attached to the first handle 7 to permit sliding along the axial direction of the wire 4. The ejector 34 can be fixed to a specific site within the sliding parameters of the first handle 7 using a screw 35. Accordingly, the position where the second handle 8 is attached to the first handle 7 can move within specific limits.

FIG. 11 is an exploded cross-sectional view of the portion of wire 4 that extends from the proximal end side of the main body 5. The wire 4 is inserted into a thin pipe 36 that consists of metal or resin, and this thin pipe 36 is then inserted into the coil shaft 37. By reinforcing with the thin pipe 36 and the coil shaft 37, the wire 4 is provided with a degree of both rigidity and flexibility as a result. Thus, the wire 4 does not bend even if the second handle 8 has been moved away from the first handle 7, while at the same time, a degree of rigidity is obtained that is sufficient to enable the wire 4 to be pushed into the main body 5 without deforming, even in the case where the treatment portion 22 is moved by advancing wire 4. Note that it is not absolutely essential to provide a thin pipe 36 as long as the desired rigidity and flexibility can be maintained. For example, a design is acceptable in which the wire 4 is inserted into just the coil shaft 37.

The action of the above-designed gripping forceps 21 during use will now be explained.

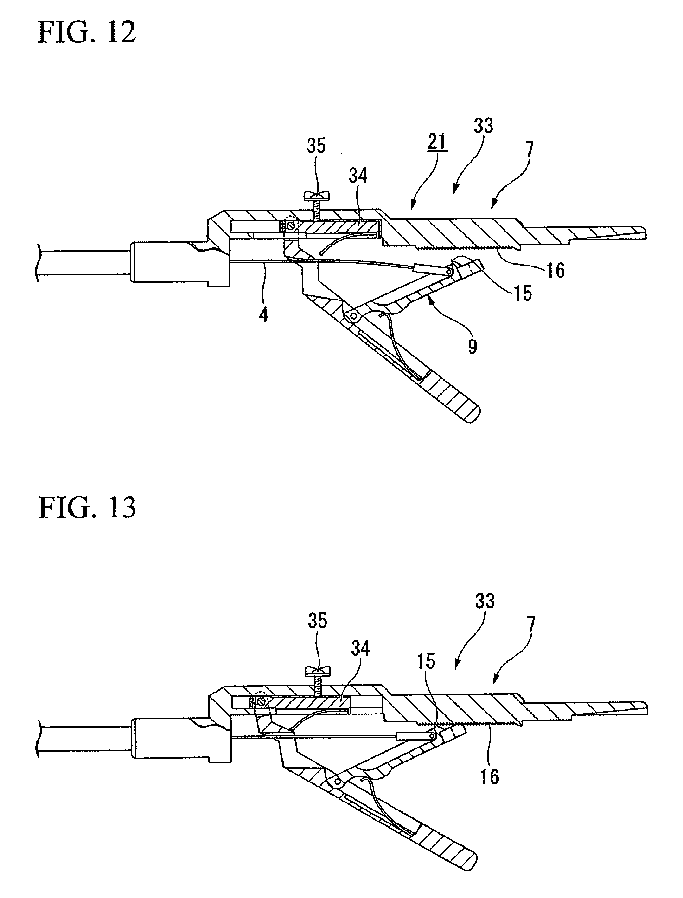

First, as shown in FIG. 12, the user moves the claw 15 of the linking member 9 away from the ratchet 16 of the first handle 7, and, in this arrangement, then adjusts the wire 4 to close the treatment portion 22. Next, the screw 35 is loosened, enabling sliding of the ejector 34 with respect to the first handle 7. In this state, the user then inserts the inserted portion 23 into the instrument channel of the endoscope.

The outer surface of the inserted portion 23 is covered with the blade 31 at this time, so that the area of contact with the inner surface of the endoscope channel becomes less as compared to a tube, etc. Accordingly, there is a decrease in the frictional force generated, thus facilitating advancing/retracting and rotating manipulation of the inserted portion 23. Further, even if friction force is generated between the inner surface of the channel and the blade 31, the resin strands that form the blade 31 move, so that the length varies between the strands. This effectively acts as a buffer for eliminating the aforementioned frictional forces to some extent.

When inserting the inserted portion 23 into the endoscope, the wire 4 is pulled toward the distal end side accompanying the spiraling, etc. of the endoscope within the body cavity. Further, as a result of the pulling force generated at this time, the ejector 34 and the second handle 8 that is attached to the ejector 34 move toward the distal end side. Accordingly, insertion of the medical treatment endoscope into the channel is prevented from becoming more difficult due to extension of the wire 4 by pulling, and contraction and hardening of the second sheath 29 and the first sheath 28 of the inserted portion 23 due to the forces opposing the pulling.

When the treatment portion 22 is projected out from the distal end of the endoscope to enable the procedure, the user fixes the ejector 34 to the first handle 7 by tightening the screw 35 as shown in the FIG 13. Then, as in the case of the needle-holding device 1 according to the first embodiment, the operator portion 33 is manipulated to open/close the treatment portion 22, the claw 15 and ratchet 16 are suitably engaged, and the desired procedure is carried out.

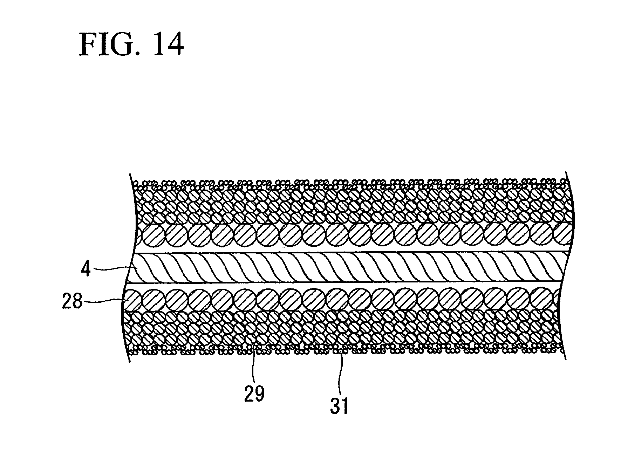

In the present embodiment, an example in which the inserted portion 23 was equipped with a cover tube 30 was explained. However, in place thereof, it is also acceptable to design an inserted portion such that the outer surface of the second sheath 29 is covered with the blade 31, and a cover tube is not provided, as in the case of the modification shown in FIG. 14.

Further, this embodiment employed an example in which the ejector 34 is fixed in place to the first handle 7 by a screw 35. However, the method for fixing the ejector to the first handle is not limited to a screw. Other examples will now be described below.

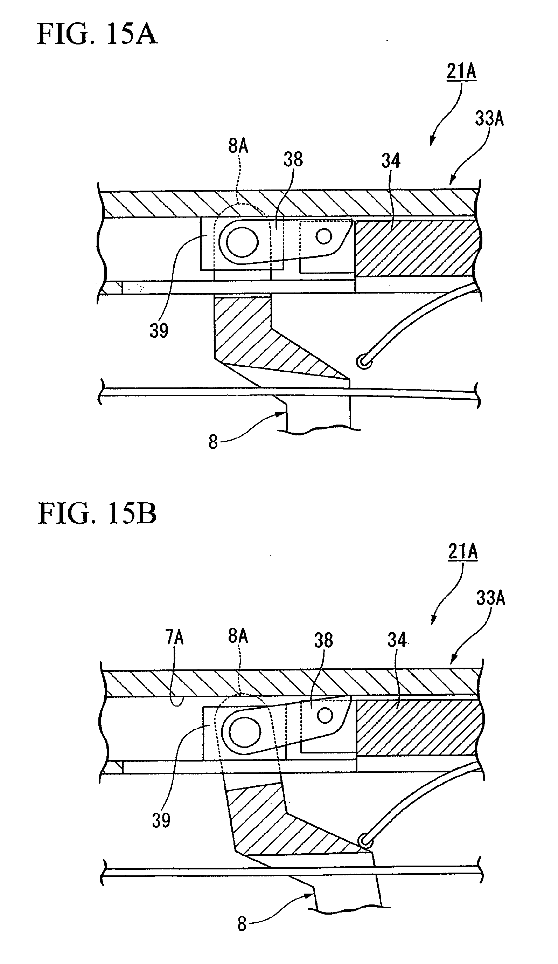

FIGS. 15A and 15B are views showing the operator portion 33A of the gripping forceps 21A according to a modification of the present embodiment. The second handle 8 is attached to the ejector 34 via a connecting member 38. In addition, a fixing member 39 is attached to the first end 8A of the second handle. As shown in FIG. 15A, in the state prior to gripping of the operator portion 33A, the top part of the connecting member 38 is roughly parallel to the axis of the first handle 7, and no friction is generated therebetween. Accordingly, the ejector 34 can slide freely with respect to the first handle 7.

When the claw 15 and the ratchet 16 are engaged as shown in FIG. 15B, the first end 8A of the second handle 8 rotates, with the coupling between the connecting member 38 and the ejector 34 serving as the fulcrum. The first end 8A moves downward, and the fixing member 39 is pushed against the lower surface of the sliding groove 7A in which the ejector 34 slides. At the same time, the proximal end side of the connecting member 38 is pushed against the top surface of the sliding groove 7A. Next, the ejector 34 is fixed in place to the first handle 7 due to the frictional force generated between the sliding groove 7A, and the fixing member 39 and the connecting member 38.

As a result of this design, the ejector 34 is fixed in place merely by gripping the first handle 7 and the second handle 8. Thus, the problem of manipulating the screw 35 is eliminated, making it possible to design a gripping forceps 21 A that is easier to operate.

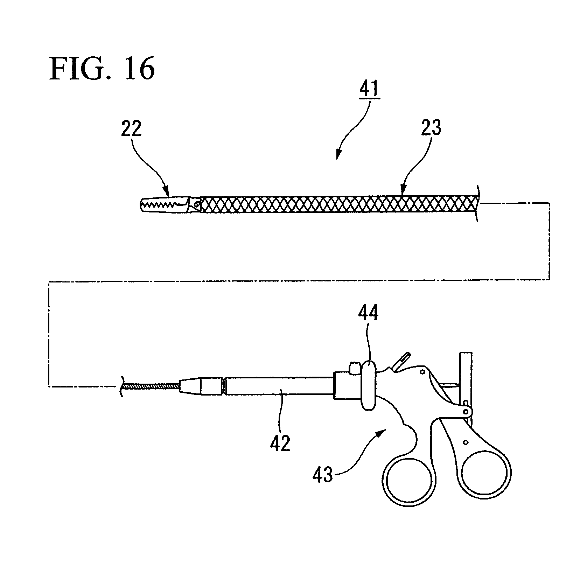

Next, a third embodiment of the present invention will be explained with reference to FIGS. 16 through 19. The endoscope treatment instrument according to this embodiment differs from the preceding endoscope treatment instrument with regard to the design of the main body and the operator portion.

Note that compositional elements that are the same as those of the above-described needle-holding device 1 and gripping forceps 21 will be assigned the same numeric symbol and a repetitive explanation thereof will be omitted.

FIG. 16 is a view showing the gripping forceps 41 that is the endoscope treatment instrument according to this embodiment The treatment portion 22 and the inserted portion 23 of the gripping forceps 41 are equivalent to those of the gripping forceps 21.

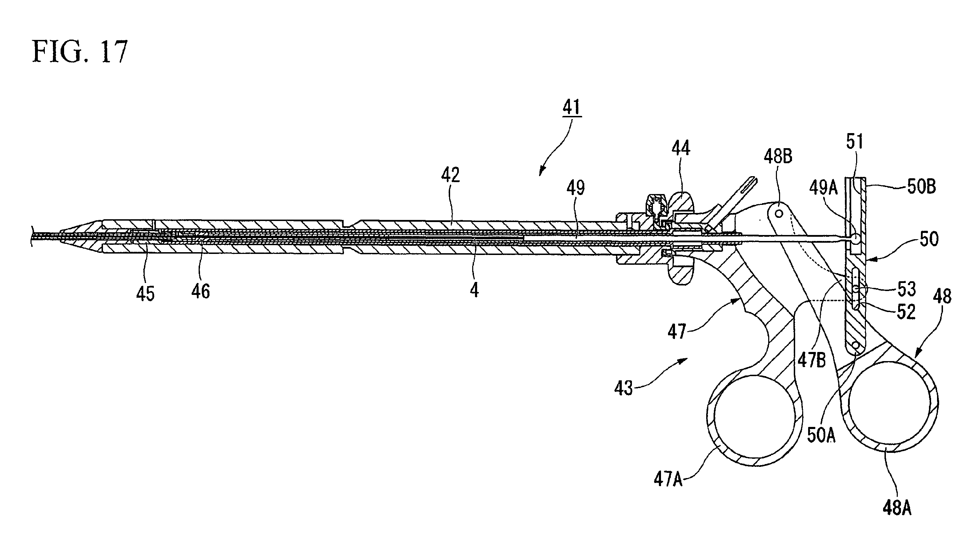

FIG. 17 is a cross-sectional view of the main body 42 and the operator portion 43. A knob 44 is attached to the proximal end side of the main body 42 for rotating the treatment portion 22. When the knob 44 is rotated, the main body 42 rotates about the axis of the wire 4. When the main body 42 rotates, a rotating member 45, which is engaged with the main body 42 at the distal end side of the main body 42, rotates. In addition, a coil connecting member 46, to which the first sheath 28 and the second sheath 29 (neither of which are shown in the figures) are connected, is engaged with the rotating member 45 in a manner which does not permit rotation. As a result, the inserted portion 23 and the treatment portion 22 rotate.

The operator portion 43 is attached in a freely rotating manner to the knob 44 at the proximal end side of knob 44. The first handle 47 and the second handle 48 each have respective rings 47A,48A for placement of the user's finger during operation, i.e., they form a so-called gun-grip type operator portion. The proximal end of the wire 4 that is inserted into the main body 42 is connected to a rod 49 inside the main body 42. The proximal end of the rod 49 extends from the first handle 47 toward the handheld side.

The first handle 47 is attached in a freely rotating manner with respect to the knob 44. The end 48B, which is the end farthest removed from the ring 48A of the second handle 48, is attached to the top part of the first handle 47 in freely rotating manner.

The first end 50A of a linking member 50 is attached near the ring 48A of the second handle 48 in a freely rotating manner. A stopping groove 51 for engaging and stopping the proximal end of the rod 49 is provided to the second end 50B side of the linking member 50. The proximal end 49A of the rod 49 is stopped in a manner which permits sliding within the stopping groove 51 and which permits rotation with respect to the linking member 50. A coupling groove 52 is provided in between the first end 50A and the stopping groove 51, and communicates with a coupler 47B that projects toward the handheld side of the first handle 47 via a pin 53 or the like. In other words, the linking member 50 can rotate within fixed limits with respect to the coupler 47B, and can slide with respect to the coupler 47B along the coupling groove 52. As a result of this design, the positional relationship between the first handle 47 and the linking member 50 can be maintained within a fixed range by the coupler 47B and the coupling groove 52.

The action of the gripping forceps 41 as designed above during use will now be explained.

First, the user inserts the inserted portion 23 into the endoscope channel with the treatment portion 22 in a closed state. The treatment portion 22 is then projected out from the distal end of the endoscope, and moved into the vicinity of the target tissue. Then, the treatment portion 22 is opened and closed by manipulating the operator portion 43, thereby carrying out the desired procedure on the target tissue.

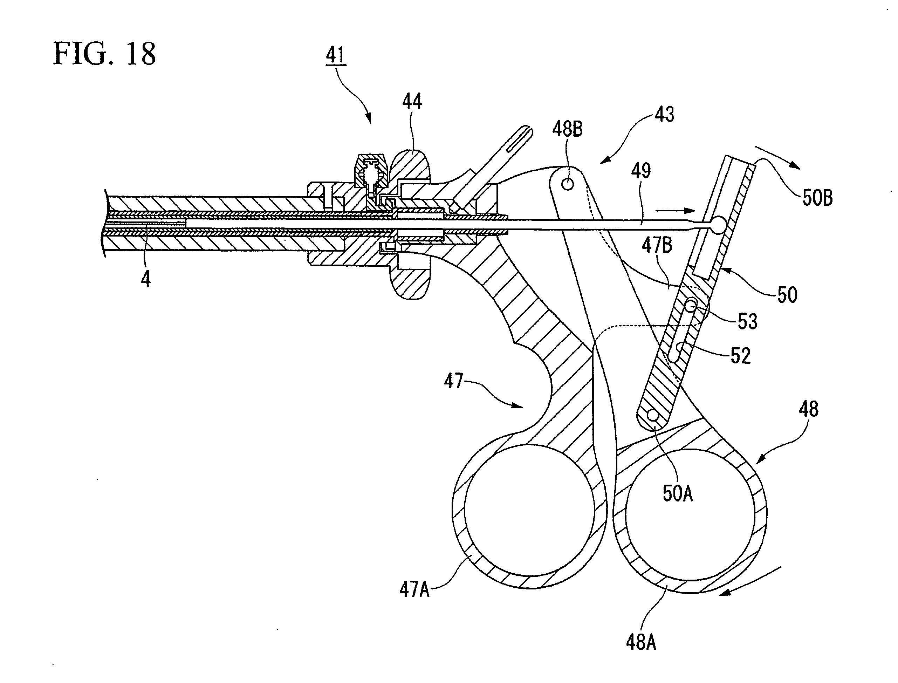

FIG. 18 is a view showing the movement of each of the sections of the operator portion 43 when an open treatment portion 22 is closed. The user rests a finger in the rings 47A and 48A, and manipulates the second handle 48 so that the ring 48A approaches the ring 47A. The second handle 48 rotates toward the distal end side, centered about the end 48B.

Accompanying the rotation of the second handle 48, the first end 50A of the linking member 50 which is coupled to the second handle moves toward the distal end side. The linking member 50 is attached so that it does not move away from the coupler 47B of the first handle 47 at this time, thus the linking member 50 rotates centered on the coupler 47B. Next, in order to increase the angle between the second handle 48 and the linking member 50 that is formed on the end 48B side, the second end 50B of the linking member 50 is moved toward the handheld side. The pin 53 which is coupled to the linking member 50 suitably slides within the coupling groove 52 so that the linking member 50 rotates smoothly at this time.

By moving the second end 50B of the linking member 50 toward the handheld side, the rod 49 and the wire 4 are pulled toward the handheld side and the treatment portion 22 is closed. When rotating the treatment portion 22, the treatment portion 22 is adjusted to the desired inclination by rotating the knob 44 with respect to the operator portion 43.

As in the case of a conventional rigid treatment instrument, the gripping forceps 41 according to the present embodiment can obtain a larger stroke as compared to the case where the proximal end 49A of the rod 49 is connected near the end 48B of the rotating second handle 48. Accordingly, even if the inserted portion 23 is flexible, operation of the treatment instrument 22 can be carried out extremely well.

While preferred embodiments of the invention have been described and illustrated above, it should be understood that these are exemplary of the invention and are not to be considered as limiting. Additions, omissions, substitutions, and other modifications can be made without departing from the spirit or scope of the present invention.

For example, the preceding embodiments described examples in which a rigid main body was provided. However, a main body is not absolutely required in the endoscope treatment instrument according to the present invention. Accordingly, when the inserted portion is employed by insertion into the channel of a typical endoscope, it is acceptable to connect the inserted portion directly to the operator portion.

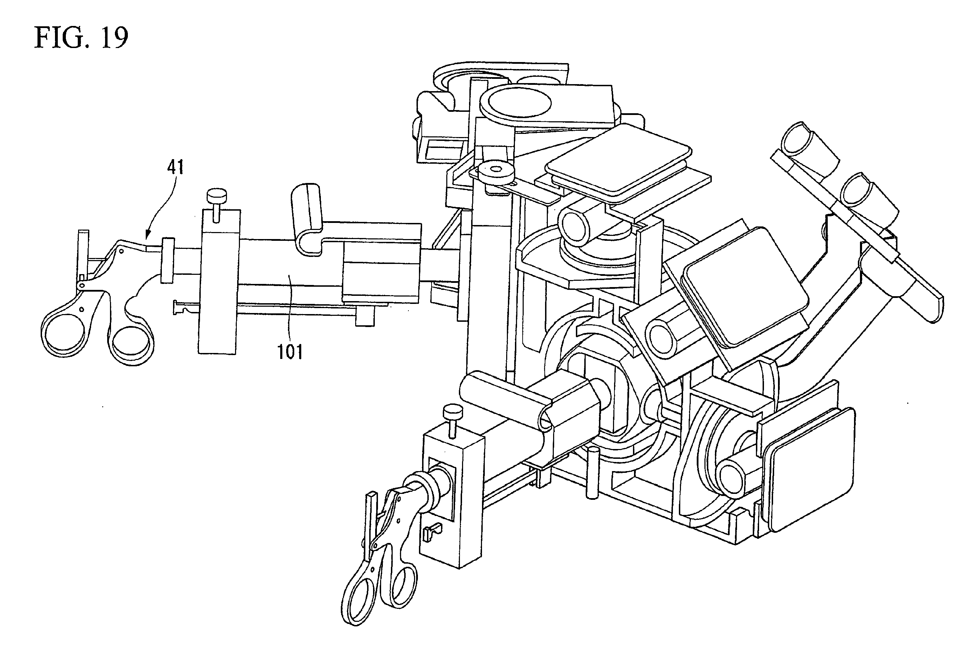

However, when the endoscope treatment instrument according to the present invention is designed equipped with a main body, as described in the preceding embodiments, then, in the above-described medical treatment endoscope, it is possible to employ the device by easily inserting it into the operation stick 101 for manipulating the arm at the distal end of the inserted portion, as shown in FIG. 19. As a result, the user can use the aforementioned medical treatment endoscope to carry out a laparoscopic procedure or other such surgical technique using a familiar inline type or gun grip type operator portion. Note that in FIG 19, the gripping forceps 41 is inserted into the operation stick 101; however, it is of course also possible to insert a needle-holding device 1 or a gripping forceps 21.

Further, the endoscope treatment instrument according to the present invention may be applied to a regular endoscope device, without providing the above-described operation stick.

Finally, the invention is not to be considered as being limited by the foregoing description, and is only limited by the scope of the appended claims.

## Claims

1. An endoscope treatment instrument (1, 21, 41) provided with:
an inserted portion (2, 23) that is flexible,
a treatment portion (3, 22) that is attached to the distal end of said inserted portion,
an operator portion (6, 33, 43) that is attached to the proximal end of said inserted portion for manipulating said treatment portion, and
a transmitting member (4) that is connected to said treatment portion and said operator portion and is inserted into said inserted portion, for transmitting the operation of said operator portion to said treatment portion; wherein:
said operator portion has a first handle (7, 47) that is attached to said inserted portion so as to prevent movement in the axial direction of said inserted portion,
a second handle (8, 48) that is attached to said first handle so as to permit rotation,
a linking member (9, 50) in which its first end (9A, 50A) is connected to said transmitting member and its second end (9B, 50B) is attached to said second handle so as to permit rotation, and
a positional relationship maintaining mechanism (15, 16, 47B, 52) which can maintain the positional relationship between said linking member and said first handle; and,
when said operator portion is operated, the angle formed by said second handle and said linking member changes, transitioning to an operating state in which said first end of said linking member has moved toward the proximal end side, and said operating state is maintained by said positional relationship maintaining mechanism.

2. An endoscope treatment instrument according to claim 1, wherein said positional relationship maintaining mechanism is composed of a claw (15) provided to said first end of said linking member, and a ratchet (16) provided to said first handle that can engage with and be released from said claw.

3. An endoscope treatment instrument according to claim 1, wherein
said transmitting member is connected to said linking member so as to permit sliding within specific limits along the length of said linking member, and
said positional relationship maintaining mechanism is composed of a groove (24A) that is provided to said linking member, and a coupling mechanism (10) that is provided to said first handle, and said linking member and said first handle are coupled so that said linking member can rotate with respect to said first handle, and said coupling mechanism can slide inside said groove.

4. An endoscope treatment instrument according to claim 1, wherein
said first handle has an ejector (34) that can slide along the axial direction of said transmitting member, and a fixing member (35) that can fix said ejector at a specific position with respect to said first handle, and
said first end of said second handle is attached to said ejector.

5. An endoscope treatment instrument according to claim 1, wherein the proximal end side of said transmitting member is flexible, and is reinforced by reinforcing members (36, 37) that has a degree of rigidity sufficient to prevent bending of said transmitting member when said transmitting member is moved toward the distal end.

6. An endoscope treatment instrument according to claim 1, wherein the outer periphery of said inserted portion is covered with a blade (31) that is formed as a net using resin strands.
